# EUROPÄISCHE PATENTSCHRIFT

(11) **EP 3 807 646 B1**
(45) Veröffentlichungstag und Bekanntmachung des Hinweises auf die Patenterteilung: **07.06.2023**
(21) Anmeldenummer: 18807568.3
(22) Anmeldetag: 12.11.2018
(51) Int. Cl.: G01N 33/68

(54) **PROBENPRÄPARATION FÜR PROTEOMISCHE UNTERSUCHUNGEN**
SAMPLE PREPARATION FOR PROTEOMIC ASSAYS
PRÉPARATION D'ÉCHANTILLONS POUR DES ÉTUDES PROTÉOMIQUES

(30) Priorität: 12.06.2018 DE 102018114028
(43) Veröffentlichungstag der Anmeldung: 21.04.2021
(73) Patentinhaber: BUNDESREPUBLIK DEUTSCHLAND letztvertreten durch das Robert Koch-Institut vertreten durch seinen Präsidenten, 13353 Berlin (DE)
(72) Erfinder: SCHNEIDER, Andy, 10319 Berlin (DE); DÖLLINGER, Jörg, 16321 Bernau (DE); LASCH, Peter, 13086 Berlin (DE)
(74) Vertreter: PATERIS Patentanwälte PartmbB
(86) Internationale Anmeldenummer: PCT/EP2018/080987
(87) Internationale Veröffentlichungsnummer: WO 2019/238255

(56) Entgegenhaltungen:
- EP-A1- 2 700 949
- US-A1- 2009 035 822
- MARTÍN JULIA ET AL: "Multi-class method for biomonitoring of hair samples using gas chromatography-mass spectrometry", ANALYTICAL AND BIOANALYTICAL CHEMISTRY, SPRINGER, DE, Bd. 407, Nr. 29, 1. Oktober 2015 (2015-10-01), Seiten 8725-8734, XP035867550, ISSN: 1618-2642, DOI: 10.1007/S00216-015-9026-2 [gefunden am 2015-10-01]
- PETER LASCH ET AL: "MALDI-TOF Mass Spectrometry Compatible Inactivation Method for Highly Pathogenic Microbial Cells and Spores", ANALYTICAL CHEMISTRY, Bd. 80, Nr. 6, 1. März 2008 (2008-03-01), Seiten 2026-2034, XP055546221, US ISSN: 0003-2700, DOI: 10.1021/ac701822j
- ALEJANDRO MIÑÁN ET AL: "Rapid identification of Burkholderia cepacia complex species including strains of the novel Taxon K, recovered from cystic fibrosis patients by intact cell MALDI-ToF mass spectrometry", THE ANALYST, Bd. 134, Nr. 6, 1. Januar 2009 (2009-01-01), Seite 1138, XP055546227, UK ISSN: 0003-2654, DOI: 10.1039/b822669e
- Joerg Doellinger ET AL: "Sample Preparation by Easy Extraction and Digestion (SPEED) - A Universal, Rapid, and Detergent-free Protocol for Proteomics based on Acid Extraction", bioRxiv, 16. August 2018 (2018-08-16), XP055546144, DOI: 10.1101/393249 Gefunden im Internet: URL:https://www.biorxiv.org/content/biorxi v/early/2018/08/16/393249.full.pdf
- Hirano ET AL: "New Protein Extraction/Solubilization Protocol for Gel-based Proteomics of Rat (Female) Whole Brain and Brain Regions", Molecules and cells, 1 August 2006 (2006-08-01), pages 119-125, XP55845523, Korea (South) Retrieved from the Internet: URL:http://www.molcells.org/journal/downlo ad_pdf.php?spage=119&volume=22&number=1 [retrieved on 2021-09-28]
- ZHONG H ET AL: "Microwave-assisted acid hydrolysis of proteins combined with liquid chromatography MALDI MS/MS for protein identification", JOURNAL OF THE AMERICAN SOCIETY FOR MASS SPECTROMETRY, ELSEVIER SCIENCE INC, US, vol. 16, no. 4, 1 April 2005 (2005-04-01), pages 471-481, XP027790406, ISSN: 1044-0305 [retrieved on 2005-04-01]

## Beschreibung

Die Erfindung betrifft ein Verfahren zur Aufbereitung biologischen Materials für proteomische Untersuchungen, wobei das Material unter Verwendung einer organischen Säure lysiert wird.

Das Proteom umfasst die Gesamtheit aller in einer Zelle oder einem Lebewesen zu einem bestimmten Zeitpunkt unter bestimmten Bedingungen exprimierten Proteine. Das Fachgebiet, welches sich mit der Analyse des Proteoms beschäftigt, wird als Proteomik bezeichnet, deren zentrale Technik derzeit die Flüssigchromatographie gekoppelt mit Massenspektrometrie (LC-MS) ist. Aufgrund der immensen Bedeutung von Proteinen für den Phänotyp von Lebewesen finden proteomische Techniken zunehmend breite Anwendung innerhalb der Medizin und der Lebenswissenschaften, unter anderem um Biomarker für Krankheiten zu identifizieren oder um zukünftig Therapieverfahren individuell an Patienten anzupassen.

Unterschiedliche Probenmaterialien wie Zellen, Gewebe oder Körperflüssigkeiten stellen dabei hohe Anforderungen an Präparationstechniken für die Proteomik, wobei einfache, schnelle, robuste und reproduzierbare Verfahren der Probenvorbereitung angestrebt werden. Diese umfassen bei der *bottom-up* Proteomik im Wesentlichen drei essentielle Schritte, (i) Lyse/Extraktion, (ii) Verdau und (iii) Peptidaufreinigung. Abhängig von der Art des Probenmaterials, der verwendeten Technik und der experimentellen Strategie kann die Probenvorbereitung um zusätzliche Schritte, wie beispielsweise Aufreinigungen oder Markierungsreaktionen, erweitert werden.

Der gegenwärtige Stand der Technik beruht auf der Extraktion von Proteinen mittels Detergenzien (z.B. Natriumlaurylsulfat (SDS), Desoxycholsäure (SDC)) und/oder chaotroper Substanzen (z.B. Harnstoff, Guanidinhydrochlorid (GnHCl)), häufig unterstützt durch physikalischen Energieeintrag (Ultraschall, Druck, Reibung, Mahlen, Hitze) um eine effektive Lyse des Probenmaterials zu erzielen.

In dem Fachartikel "Multi-class method for biomonitoring of hair samples using gas chromatography-mass spectrometry" (Martin Julia et al., 2015, Analytical and bioanalytical chemistry 407: 8725-9734) wird ein Verfahren zur Untersuchung von menschlichen Haarproben zur Bestimmung von verschiedenen chemischen Klassen neuer Schadstoffe mittels GC-MS beschrieben. Die dabei verwendete Präparationstechnik beinhaltet die Behandlung menschlicher Haare über Nacht mit Trifluoressigsäure in Methanol (1.5:8.5, v/v), einer Flüssig-Flüssig-Extraktion mit Hexan/Ethylacetat zum Zwecke der proteolytischen Spaltung ("Hydrolyse") von Keratin, welche mit proteomischen LC-MS Untersuchungsmethoden unvorteilhaft interferieren würde, gefolgt von einer Extraktion/Separation der Analyten für die GC-MS Analytik.

Die Druckschrift EP 2 700 949 A1 bezieht sich auf eine proteomische Untersuchung von Tumorgewebe. Dabei wird das Probenmaterial (Tumorgewebe) durch Ultraschall kombiniert mit einem Gefrier-Tau-Zyklus mechanisch aufgeschlossen. Der Ansatz wird dabei bei 0°C (Eiswasserbad) inkubiert, und der Probenpuffer umfasst zur leichten Ansäuerung 0.1% einer organischen Säure - d.h. Säuren in einer tausendfachen Verdünnung. Nicht aufgeschlossene Probenbestandteile müssen im Anschluss zentrifugiert und der erhaltene Überstand entnommen werden

In der Druckschrift US 2009/035822 A1 wird mittels ELISA die Konzentration einer bestimmten Substanz (Substanz P) in Zellen bestimmt, die zu diesem Zweck durch nacheinander folgende Behandlungen beginnend mit zwei unterschiedlichen komplexen Salzlösungen, später dann mit einer Kombination aus stark verdünnter Essigsäure und Trifluoressigsäure, aufgeschlossen, jedoch nicht vollständig lysiert werden.

In dem Fachartikel "MALDI-TOF Mass Spectrometry Compatible Inactivation Method for Highly pathogenic Microbial Cells and Spores" (Lasch et al., 2008, Analytical Chemistry 80: 2026-2034) wird ein Verfahren zu Inaktivieren von Bakterien, inklusive bakterieller Endosporen der Biosicherheitsstufe 3 beschrieben, das für eine nachfolgenden taxonomische Analyse (Identifikation auf Spezies-Ebene) mittels MALDI-ToF-Massenspektrometrie geeignet ist.

In dem Fachartikel "Rapid Identification of Burkholderia cepacia complex species including strains of the novel Taxon K, recovered form cystic fibrosis patients by intact cell MALDI-ToF mass spectrometry" (Minan et al., 2009, The Analyst 134: 1138) wird die Anwendung von MALDI-ToF-Massenspektrometrie zur Identifizierung von Bakterien des Burkholderia cepacia complex beschrieben, wobei das Verfahren von Lasch et al. verwendet wird.

In dem Fachartikel "New Protein Extraction/Solubilization Protocol for Gelbased Proteomics of Rat (Female) Whole Brain and Brain Regions" (Hirano et al., 2006, Molecules and Cells, S. 119-125) wird biologisches Material durch mechanische Einwirkung zerkleinert. Nach der Zerkleinerung des Materials werden Proteine durch eine Inkubation mit einem Aceton-TCA-Gemisch bei - 20°C gefällt.

In dem Fachartikel "Microwave-assisted acid hydrolysis of proteins combined with liquid chromatography MALDI MS/MS for protein identification" (Zhong et al., 2005, Journal of the American Society for Mass Spectrometry 16: 471-481) wird eine Mikrowellen-assistierte Hydrolyse von Proteinen beschrieben. Diese werden entweder bereits gereinigt bereitgestellt oder aufwändig aufgereinigt. Insbesondere bei schwer aufschließbaren Proben (z.B. Gewebe mit hohem Kollagenanteil) sind häufig weitere Arbeitsschritte oder der Einsatz zusätzlicher Reagenzien nötig. Die Anwesenheit dieser Reagenzien kann sich jedoch negativ auf die Effizienz nachfolgender Präparationsschritte auswirken (Verdau), so dass Standardprotokolle der *bottom-up* Proteomik vielfach weitere Prozeduren zur Entfernung von Detergenzien, chaotroper Substanzen und ähnlicher Wirkstoffe enthalten. Die zusätzlichen Arbeitsschritte wirken sich dabei generell negativ auf Reproduzierbarkeit und Proteinausbeute aus. Vor dem Hintergrund des großen Potentials der Proteomik, beispielsweise im Rahmen der personalisierten Medizin, wird jedoch eine einfache, direkte, reproduzierbare und automatisierbare Methodik der Probenpräparation angestrebt.

Diese Aufgabe wird durch ein erfindungsgemäßes Verfahren mit den Merkmalen von Anspruch 1 gelöst. Weitere vorteilhafte Ausführungsformen und Ausgestaltungen der Erfindung ergeben sich aus den Neben- und Unteransprüchen, den Figuren und den Ausführungsbeispielen. Die Ausführungsformen der Erfindung sind in vorteilhafter Weise miteinander kombinierbar. Für einen Fachmann naheliegende Abwandlungen und Änderungen der Erfindung fallen unter den Schutzumfang der Patentansprüche.

Ein erster Aspekt der Erfindung betrifft ein Verfahren zum Aufschluss einer Probe biologischen Materials zur Proteomanalyse durch ein massenspektrometrisches Verfahren, welches folgende Arbeitsschritte umfasst:
- Bereitstellen der Probe in einem Reaktionsgefäß, wobei das biologische Material Proben menschlichen, tierischen oder pflanzlichen Ursprungs umfasst,
- Zugabe einer bestimmten Menge einer organischen Säure zu der Probe in einem Verhältnis, dass sich die Probe vollständig löst,
- Inkubation des Ansatzes bei Raumtemperatur,
- Zugabe einer neutralisierenden Lösung zum Ansatz, bis ein pH-Wert zwischen 7 und 9 erreicht wird.

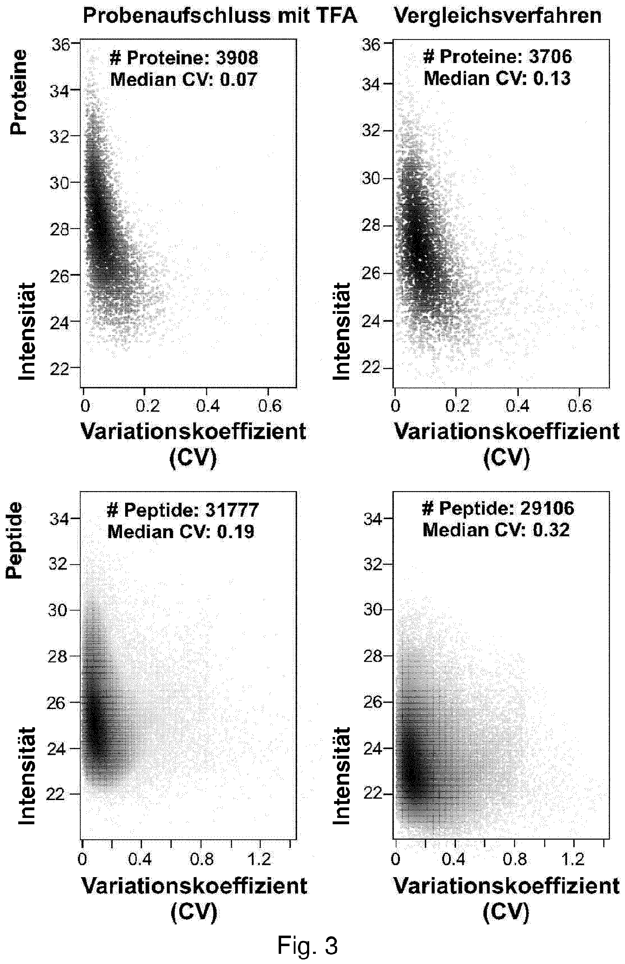

Das erfindungsgemäße Verfahren ist vorteilhaft, weil im Vergleich zu herkömmlichen Verfahren weniger Arbeitsschritte benötigt werden. Damit ist es zeitlich und vom Arbeitsaufwand ökonomischer als herkömmliche Verfahren. Weiterhin werden mit erfindungsgemäß aufgeschlossenem Probematerial verglichen mit herkömmlichen Verfahren vorteilhafterweise eine größere Intensität der Signale mit einer geringeren Varianz erhalten (Fig. 3). Ein weiterer Vorteil liegt in einer hohen Reproduzierbarkeit des Verfahrens, was besonders in Bezug auf quantitative Analysen wesentlich ist. Darüber hinaus sind Proteine in derartigen Lösungen mit hoher Salzkonzentration sehr stabil, was Transport, Lagerung und Archivierung der Proben erheblich erleichtert.

Zudem ist das Verfahren vorteilhafterweise für jede Art von biologischem Material verwendbar, das Proteine enthält, die proteomisch analysiert werden sollen. Vorzugsweise umfasst das biologische Material Proben menschlichen, tierischen oder pflanzlichen Ursprungs, insbesondere Gewebe, Zellen, Körperflüssigkeiten, Blut- und Blutprodukte, Abstriche sowie Fäzes.

Das biologische Material wird in einem Reaktionsgefäß aus einem geeigneten Material und in geeigneter Größe bereitgestellt. Vorteilhaft sind Gefäße aus Plastik mit einem Volumen von bis zu 2 ml, z.B. im Laborbereich häufig verwendete sogenannte Mikroreaktionsgefäße.

Die Menge der zuzugebenden organischen Säure liegt im Ermessen des Fachmanns. Für eine bekannte Menge an biologischem Material wird idealerweise eine standardisierte Menge an Säure zugegeben. Idealerweise wird ein Probe-zu-Säure Verhältnis derart eingestellt, dass sich das Probenmaterial vollständig löst.

Das Probenmaterial kann mit der Säure ohne weiteres bis zu 60 min bei Raumtemperatur inkubiert werden, ohne dass die Qualität der Proteine leidet. Dabei kann die Lyse beendet werden, sobald die Suspension klar wird, was bei den meisten Proben nach 2-10 Min der Fall ist.

Die Neutralisation der Probe erfolgt, um die Proteine für den Verdau vorzubereiten. Zum Neutralisieren wird eine schwache Base verwendet, z.B. eine wässrige 2M-TRIS-Lösung (Tris(hydroxymethyl)-aminomethan), wobei das Volumen der TRIS-Lösung bei etwa dem 7-15 fachen des Probenvolumens liegen soll. Es wird dabei ein pH-Wert angestrebt, der zwischen 7 und 9, idealerweise zwischen 8 und 8,5 liegt.

Vorzugsweise umfasst das Verfahren einen zusätzlichen Schritt einer elektromagnetischen Bestrahlung direkt vor der Neutralisation. Dieser Schritt ist besonders vorteilhaft für schwer aufschließbare Proben, beispielsweise Proben mit hohem Kollagenanteil. Eine thermische Behandlung bei Temperaturen >40°C zeigt vergleichbare Effekte.

Besonders bevorzugt wird die Probe durch Mikrowellen bestrahlt. Dabei wird die Probe für einen Zeitraum von 5-30 s, besonders für 10 s, einer Mikrowellenleistung von 800 W ausgesetzt. Es ist vorteilhaft, das Reaktionsgefäß, in dem die Probe inkubiert ist, mit einer zusätzlichen Umhüllung, z.B. einem weiteren Gefäß aus Plastik, zu umgeben. Es hat sich herausgestellt, dass die Mikrowellenbehandlung für jede Art von biologischem Material, insbesondere jedoch für schwer zu lysierendes Material, vorteilhaft für eine effektive Lyse ist.

Bevorzugt wird in dem Verfahren als organische Säure eine Karbonsäure verwendet. Noch bevorzugter wird in dem Verfahren eine Halogen-Karbonsäure verwendet. Besonders bevorzugt wird in dem Verfahren als organische Säure Trifluoressigsäure (TFA) und deren Derivate verwendet. Es hat sich gezeigt, dass TFA besonders effektiv in der Lyse von Geweben und Zellen ist. Dabei ist die Verwendung von TFA auch vorteilhaft, weil die Analyse der Proteine nicht durch die Wirkung der TFA beeinträchtigt wird.

Weiterhin umfasst das Verfahren vorzugsweise die weiteren Schritte:
- Zugabe einer Alkylierungslösung,
- Zugabe einer Reduktionslösung,
- Bestimmen der Proteinkonzentration,
- Verdünnen der Probe mit Wasser,
- Enzymatische oder chemische Spaltung der Proteine,
- Aufreinigung der generierten Peptide.

Ein zweiter Aspekt der Erfindung betrifft eine Verwendung einer durch das erfindungsgemäße Verfahren vorbereiteten Probe biologischen Materials in einem massenspektrometrischen Verfahren.

Die Vorteile der besagten Verwendungen entsprechen den Vorteilen des erfindungsgemäßen Verfahrens.

Die Erfindung wird anhand der Figuren näher erläutert. Es zeigen
- Figur 1: ein Fließdiagramm einer Ausführungsform des erfindungsgemäßen Verfahrens.
- Figur 2: eine vergleichende Darstellung der Lyse von Proben.
- Figur 3: Ergebnisse einer Proteomanalyse humaner Zellen des beschriebenen Verfahrens im Vergleich mit einer Referenzmethode (Triplikate).

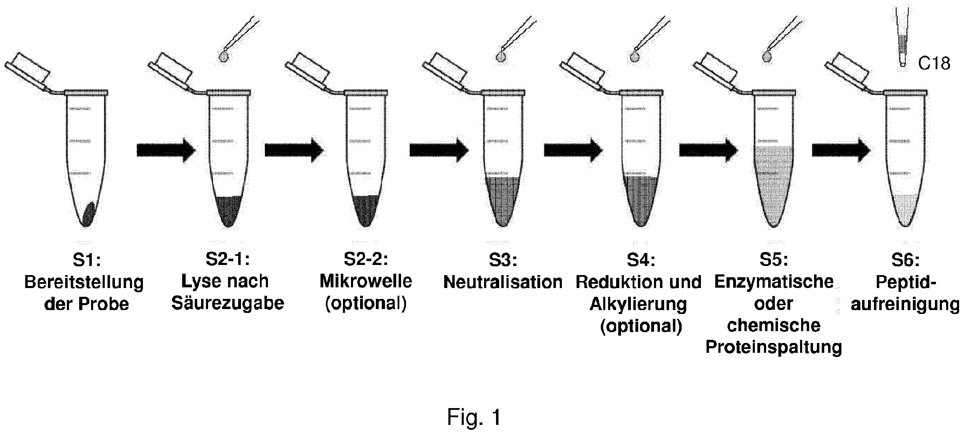

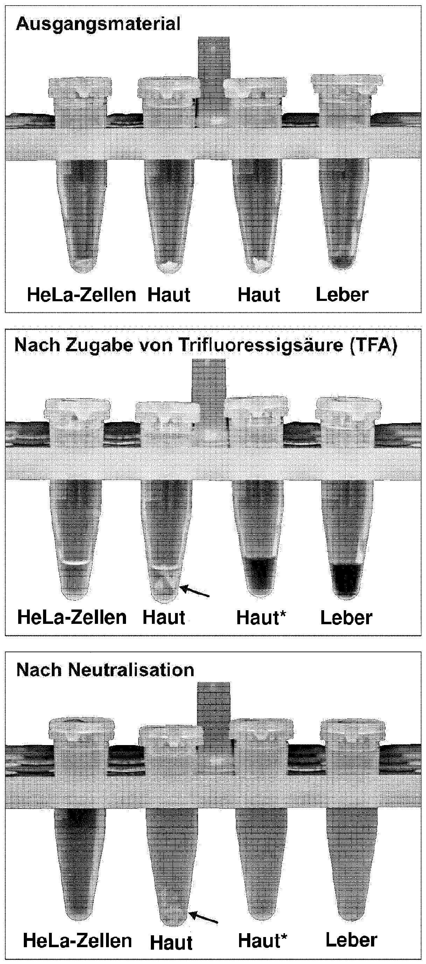

In einer Ausführungsform des erfindungsgemäßen Verfahrens gemäß der Darstellung von Fig. 1 wird in einem ersten Schritt S1 eine Probe eines biologischen Materials bereitgestellt. Das biologische Material kann eine Gewebeprobe sein, z.B. eine Blutprobe, oder eine Suspension oder ein Pellet von Zellen sowie feste Gewebestrukturen.

In einem zweiten Schritt S2-1 wird reine Trifluoressigsäure im Überschuss dem biologischen Material zugegeben, idealerweise in einem

Volumenverhältnis zwischen 1:1-1:10 (Probe zu TFA). Der Ansatz wird gemischt, z.B. mittels eines Vortex-Gerätes, und bei Raumtemperatur für 1-10 min inkubiert. Das Probenmaterial wird dabei lysiert. Neben dem zeitlichen Ablauf wird der Lyse-Verlauf visuell verfolgt; wenn die Lösung klar wird, ist das Probenmaterial lysiert worden (Fig. 2). Ist die Proteinkonzentration zu hoch, um mit der angegeben Säuremenge extrahiert zu werden, wird ein angemessenes weiteres Volumen an Säure hinzugefügt, z.B. 100 µl. In einem optionalen Schritt S2-2, der den Aufschluss schwer zu lysierenden Materials unterstützt, wird das Material für 10 s bei einer Leistung von 800 W in einem Mikrowellengerät inkubiert.

Zur Illustration der Säurewirkung auf die Proben werden in Fig. 2 verschiedene Probenarten dargestellt, jeweils vor und nach der Lyse. Von links nach rechts sind dargestellt: eine Suspension von HeLa-Zellen (Mensch), zwei Hautproben (Huhn) sowie eine Leberprobe (Huhn). Es werden jeweils das Ausgangsmaterial, sowie die Proben nach Lyse mit TFA bzw. nach erfolgter Neutralisation dargestellt. Während die HeLa -Zellen sowie das Lebergewebe durch alleinige Säurezugabe vollständig lysiert werden, zeigen sich nach Zugabe von TFA bzw. nach Neutralisation in einer der Hautproben nicht aufgelöste Gewebebestandteile (zweite Probe von links, siehe Pfeile). Diese Bestandteile können aufgelöst werden, wenn die Probe mit Mikrowellen bestrahlt wird (Probe Haut*, siehe dritte Probe von links).

Nach Klären der Lösung wird der Ansatz des lysierten Materials in einem dritten Schritt S3 mit einer wässrigen 2M TRIS-Lösung neutralisiert. Dabei wird zum Neutralisieren eines Volumens der Probe etwa das 8-15-fache Volumen einer 2M-TRIS-Lösung hinzugefügt, d.h. für 100 µl Lyse-Ansatz werden 1000 µl an 2M TRIS-Lösung hinzugefügt.

In jedem Fall wird in Schritt S3 der pH-Wert der Lösung kontrolliert. Es wird dabei ein pH-Wert angestrebt, der zwischen 7 und 9, idealerweise zwischen 8 und 8,5 liegt. Zum Erreichen des angestrebten pH-Wertes wird ggf. durch Zugabe von 2M TRIS oder von einer verdünnten Trifluoressigsäure-Lösung nachgesteuert, bis der optimale pH-Wert erreicht ist.

Nach dem Neutralisieren wird die Lösung in einem vierten Schritt S4 mit einer Reduktions- und Alkylierungslösung versetzt. Dazu wird eine frisch angesetzte Lösung von 100 mM TCEP (Tris(2-Carboxyethyl)phosphin) und 400 mM CAA (Chloracetamid) in Wasser verwendet, von der 10% des Probenvolumens zur Probe hinzugefügt werden. Die Proben werden für einen Zeitraum von mindestens 3-10 min bei 95°C in einem Thermomixer inkubiert. Nach diesem Schritt können die Proben eingefroren und über einen längeren Zeitraum bei Temperaturen ≤ -20°C gelagert werden. Der Schritt S4 kann entfallen, wenn Peptide analysiert werden, die keine Cysteine enthalten. Es können auch alternative Reduktions- und Alkylierungsreagenzien, wie z.B. DTT (Dithiothreitol) oder IAA (Iodacetamid) verwendet werden, wobei die Inkubationsbedingungen entsprechend anzupassen sind.

In einem fünften Schritt S5 wird das Verfahren fortgeführt, indem die Proteine der Probe enzymatisch oder chemisch in Peptide gespalten werden. Für einen enzymatischen Verdau wird dazu auf eine dem Fachmann bekannte Weise die Proteinkonzentration der Probe bestimmt, und anschließend Trypsin oder ein anderes geeignetes Enzym in einem definierten Massenverhältnis, bei Trypsin im Bereich zwischen 500:1 bis 5:1 (Protein: Trypsin), zur Probe gegeben. Die Probe kann mit Wasser verdünnt werden je nach Abhängigkeit der Aktivität des verwendeten Enzyms von der Pufferkonzentration des Lysats. Der Verdauansatz wird anschließend bei einer für das Enzym optimalen Temperatur (37°C für Trypsin) für eine geeignete Zeit (1-24h für Trypsin) inkubiert.

In einem sechsten Schritt S6 werden die Peptide aufgereinigt, z.B. durch eine Festphasenextraktion an C18-Material.

Die gereinigten Peptide können anschließend mittels Flüssigchromatographie gekoppelt mit Massenspektrometrie (LC-MS) oder mit vergleichbaren Methoden wie Kapillarelektrophorese gekoppelt mit Massenspektrometrie analysiert werden. In Fig. 3 ist ein Vergleich der Ergebnisse einer Proteomuntersuchung humaner Zellen mit einer alternativen Probenpräparationsmethode dargestellt. Hierfür wurde der Koeffizient der Varianz (CV) gegenüber der Signalintensität für Peptide und für die resultierenden Proteine in einem Scatterplot dargestellt. Die Punktwolke wurde mit einer HeatMap-Darstellung überlagert. Die Anzahl der quantifizierten Peptide und Proteine sowie die Mittelwerte der CVs sind ebenfalls angegeben. Auf der linken Seite des Diagramms sind Ergebnisse dargestellt, die mit dem erfindungsgemäßen Verfahren gemäß Fig. 1 aufgeschlossen wurden, und die Diagramme auf der rechten Seite zeigen Ergebnisse einer mit herkömmlichen Verfahren aufgeschlossen Probe. Es ist zu erkennen, dass mit dem erfindungsgemäßen Verfahren mehr Peptide und daraus abgeleitet mehr Proteine bei einer geringeren Signalvarianz erfasst werden können als mit einem herkömmlichen Verfahren.

## Patentansprüche

1. Verfahren zum Aufschluss einer Probe biologischen Materials zur Proteomanalyse durch ein massenspektrometrisches Verfahren, umfassend die Schritte:
- Bereitstellen der Probe in einem Reaktionsgefäß, wobei das biologische Material Proben menschlichen, tierischen oder pflanzlichen Ursprungs umfasst,
- Zugabe einer bestimmten Menge einer organischen Säure zu der Probe in einem Verhältnis, dass sich die Probe vollständig löst,
- Inkubation des Ansatzes bei Raumtemperatur,
- Zugabe einer neutralisierenden Lösung zum Ansatz, bis ein pH-Wert zwischen 7 und 9 erreicht wird.

2. Verfahren nach Anspruch 1, wobei das biologische Material Gewebe, Zellen, Körperflüssigkeiten, Blut- und Blutprodukte, Abstriche sowie Fäzes umfasst.

3. Verfahren nach einem der vorherigen Ansprüche, wobei das Verfahren den zusätzlichen Schritt einer elektromagnetischen Bestrahlung oder des Erhitzens des Probenansatzes umfasst.

4. Verfahren nach Anspruch 3, wobei als Bestrahlung eine Mikrowellenbestrahlung durchgeführt wird oder die Probe auf über 40°C erhitzt wird.

5. Verfahren nach einem der vorherigen Ansprüche, wobei als organische Säure eine Karbonsäure verwendet wird.

6. Verfahren nach einem der vorherigen Ansprüche, wobei als organische Säure eine Halogen-Karbonsäure verwendet wird.

7. Verfahren nach einem der vorherigen Ansprüche, wobei als organische Säure Trifluoressigsäure und deren Derivate verwendet werden.

8. Verfahren nach einem der vorherigen Ansprüche, umfassend die weiteren Schritte:
- Zugabe einer Alkylierungslösung,
- Zugabe einer Reduktionslösung,
- Bestimmen der Proteinkonzentration
- Verdünnen der Probe mit Wasser,
- Enzymatische oder chemische Spaltung der Proteine,
- Aufreinigung der generierten Peptide.

9. Verwendung einer durch ein Verfahren gemäß einem der Ansprüche 1-8 vorbereiteten Probe biologischen Materials in einem massenspektrometrischen Verfahren.

## Claims

1. Method for disrupting a sample of biological material for proteome analysis by a mass spectrometry method, comprising the steps of:
- providing the sample in a reaction vessel, the biological material comprising samples of human, animal or plant origin,
- adding a particular amount of an organic acid to the sample in a ratio such that the sample dissolves completely,
- incubating the mix at room temperature,
- adding a neutralizing solution to the mix until a pH between 7 and 9 is attained.

2. Method according to Claim 1, wherein the biological material encompasses tissues, cells, body fluids, blood and blood products, swabs and faeces.

3. Method according to either of the preceding claims, wherein the method comprises the additional step of electromagnetic irradiation or heating of the sample mix.

4. Method according to Claim 3, wherein the irradiation carried out is microwave irradiation or the sample is heated to above 40°C.

5. Method according to any of the preceding claims, wherein the organic acid used is a carboxylic acid.

6. Method according to any of the preceding claims, wherein the organic acid used is a halogenated carboxylic acid.

7. Method according to any of the preceding claims, wherein the organic acid used is trifluoroacetic acid and derivatives thereof.

8. Method according to any of the preceding claims, comprising the further steps of:
- adding an alkylation solution,
- adding a reduction solution,
- determining the protein concentration,
- diluting the sample with water,
- enzymatically or chemically cleaving the proteins,
- purifying the peptides generated.

9. Use of a sample of biological material prepared by a method according to any of Claims 1-8 in a mass spectrometry method.

## Revendications

1. Procédé de décomposition d'un échantillon de matière biologique en vue d'une analyse protéomique par un procédé de spectrométrie de masse, ledit procédé de décomposition comprenant les étapes suivantes :
- fournir l'échantillon dans une cuve de réaction, la matière biologique comprenant des échantillons d'origine humaine, animale ou végétale,
- ajouter une quantité déterminée d'un acide organique à l'échantillon dans une proportion telle que l'échantillon se dissout complètement,
- laisser la préparation incuber à la température ambiante,
- ajouter une solution neutralisante à la préparation jusqu'à ce qu'un pH compris entre 7 et 9 soit atteint.

2. Procédé selon la revendication 1, la matière biologique comprenant des tissus, des cellules, des fluides corporels, du sang et des produits sanguins, des écouvillons et des matières fécales.

3. Procédé selon l'une des revendications précédentes, le procédé comprenant l'étape supplémentaire d'irradiation électromagnétique ou de chauffage de la préparation d'échantillon.

4. Procédé selon la revendication 3, l'irradiation effectuée étant une irradiation par micro-ondes ou l'échantillon étant chauffé à plus de 40 °C.

5. Procédé selon l'une des revendications précédentes, l'acide organique utilisé étant un acide carboxylique.

6. Procédé selon l'une des revendications précédentes, l'acide organique utilisé étant un acide carboxylique halogéné.

7. Procédé selon l'une des revendications précédentes, l'acide organique utilisé étant l'acide trifluoroacétique et ses dérivés.

8. Procédé selon l'une des revendications précédentes, comprenant les étapes supplémentaires suivantes :
- ajouter une solution d'alkylation,
- ajouter une solution de réduction,
- déterminer la concentration en protéines,
- diluer l'échantillon avec de l'eau,
- effectuer un clivage enzymatique ou chimique des protéines,
- purifier les peptides produits.

9. Utilisation d'un échantillon de matière biologique préparé par un procédé selon l'une des revendications 1 à 8 dans un procédé de spectrométrie de masse.
